# EUROPEAN PATENT APPLICATION

(11) **EP 4 094 705 A2**
(43) Date of publication of application: **30.11.2022**
(21) Application number: 22175845.1
(22) Date of filing: 27.05.2022
(51) Int. Cl.: A61B 34/00, A61B 34/30, A61B 1/00, A61B 90/00

(54) **TENDON-SHEATH DRIVING APPARATUS, SURGICAL MEMBER DRIVING APPARATUS, AND METHOD OF OPERATING THE SAME**

(30) Priority: 28.05.2021 KR 20210068995
(71) Applicant: Endo Robotics Co., Ltd., Seoul 02841 (KR)
(72) Inventor: KIM, Byung Gon, Seoul 02583 (KR); KIM, Kyungnam, Seoul 02858 (KR)
(74) Representative: Maiwald GmbH

(57) **Abstract**

Disclosed is a surgical member driving apparatus. According to an embodiment of the disclosure, a surgical member driving apparatus for operating a surgical member detachably mounted to an endoscope includes a cartridge module including a base unit fastened to one end portion of the endoscope and coupling with the surgical member at one side, a wire including a first end portion coupled to the base unit and transmitting driving force to the base unit, and a cartridge provided with a power transmitter coupled to a second end portion of the wire; and a driving module including an accommodating portion to which the cartridge is detachably coupled, and providing the driving force to the wire of the cartridge module coupled to the accommodating portion.

## Description

### CROSS REFERENCE TO RELATED APPLICATION

The present application claims priority to Korean Patent Application No. 10-2021-0068995, filed May 28, 2021, the entire contents of which is incorporated herein for all purposes by this reference.

### BACKGROUND OF THE INVENTION

### Field of the Invention

The disclosure relates to a tendon-sheath driving apparatus, a surgical member driving apparatus, and a method of operating the same, and more particularly to a tendon-sheath driving apparatus detachably coupled to a driving module, and a surgical member driving apparatus including a base unit detachably installed in an endoscope.

### Description of the Related Art

Surgical robots have been being developed to operate in areas where a doctor's view or a manual surgical instrument is hard to reach. To insert such surgical robots through body parts, a robot arm is required to be miniaturized.

Meanwhile, a surgical robot essentially needs an endoscope to secure a view inside a human body.

However, endoscope equipment distributed in the existing medical profession is relatively expensive, and its use is very familiar to medical personnel because the endoscope equipment has been widely used in the medical profession for many years. Taking this into account, it may be advantageous to introduce a surgical robot using an endoscope system in terms of economy and user experience.

When a robot arm is added to the endoscope, an additional control device for actuating the robot arm is needed in addition to an endoscope control device. Further, it may not be easy to replace the robot arm after one operation because the robot arm needs many wires connected to an actuator unlike the endoscope.

Accordingly, it is required to develop a surgical member driving apparatus including the robot arm detachably installed in the endoscope, thereby having advantages in terms of the economy and the user experience while solving the above problems.

### Documents of Related Art

(Patent Document 0001) Korean Patent Publication No. 10-2017-0070604

### SUMMARY OF THE INVENTION

An embodiment of the disclosure is to provide a tendon-sheath driving apparatus detachably coupled to a separate driving module.

An embodiment of the disclosure is to provide a surgical member driving apparatus detachably coupled to an endoscope system.

An embodiment of the disclosure is to provide a surgical member driving apparatus in which a consumable robot arm is easily replaceable.

An embodiment of the disclosure is to provide a surgical member driving apparatus that is easily controllable by an operator.

An embodiment of the disclosure is to provide a surgical member driving apparatus that is controllable together with an endoscope by one operator.

According to an aspect of the disclosure, there is provided a surgical member driving apparatus for operating a surgical member detachably mounted to an endoscope, including: a cartridge module including a base unit fastened to one end portion of the endoscope and coupling with the surgical member at one side, a wire including a first end portion coupled to the base unit and transmitting driving force to the base unit, and a cartridge provided with a power transmitter coupled to a second end portion of the wire; and a driving module including an accommodating portion to which the cartridge is detachably coupled, and providing the driving force to the wire of the cartridge module coupled to the accommodating portion.

In this case, the base unit may include a base portion to be fastened to the one end portion of the endoscope; and a first rotary portion rotatably coupled to the base portion with respect to an extending direction of the endoscope.

In this case, the wires may form a pair, and the pair of wires may include a first wire and a second wire to rotate the first rotary portion clockwise or counterclockwise with respect to the extending direction of the endoscope.

In this case, the surgical member driving apparatus may further include an arm portion extended in the extending direction of the endoscope, and coupled to the first rotary portion pivotally with respect to a first rotary axis perpendicular to the extending direction of the endoscope, and a third wire and a fourth wire to pivot the arm portion around the first rotary axis.

In this case, the arm portion may include a fastening member to fasten the surgical member to one side of the arm portion.

In this case, the surgical member driving apparatus may further include a second rotary portion coupled to an end portion of the arm portion pivotally with respect to a second rotary axis perpendicular to the extending direction of the arm portion.

In this case, the surgical member may include forceps provided in the second rotary portion pivotally with respect to the second rotary axis and including a first gripper and a second gripper.

In this case, the second rotary portion may include a fastening member to fasten the surgical member to the second rotary portion.

In this case, the surgical member may include one among surgical forceps, an endoscopic submucosal dissection (ESD) knife, a surgical snare, a surgical basket, a surgical net, a surgical clip, surgical micro-scissors.

In this case, the driving module may include a holding unit to mechanically hole the cartridge seated in the accommodating portion to the accommodating portion.

In this case, the surgical member driving apparatus may further include a control module to input a control command for controlling operations of the surgical member or the base unit through the driving module.

In this case, the control module may include a body; and a control stick or a control button pivotally provided on the body, and the wire may be actuated as the control stick is pivoted or the control button is pressed.

According to another aspect of the disclosure, there is provided a tendon-sheath driving apparatus for driving a tendon and a sheath including: a supporting member to which at least one side of the sheath is fastened; a movable member coupled to one side of the tendon to move the tendon relative to the sheath; and a driving module mechanically coupled to the movable member and moving or rotating the movable member, wherein the movable member is detachably coupled to the driving module.

In this case, the driving module may include a linear actuator coupled to the movable member and making the movable member move rectilinearly, and the tendon may move relative to the sheath by the rectilinear movement of the movable member.

In this case, the supporting member and the movable member may be formed as a single body to be replaced and discarded after one or more uses.

In this case, the tendon may include a plurality of tendons, the supporting member and the movable member may respectively include a plurality of supporting members and a plurality of moveable members to correspond to the plurality of tendons, and the plurality of movable members may be installed in a cartridge shaped like an enclosure, and integrally coupled to the driving module.

In this case, the cartridge may include a guide portion at one side thereof to guide the movable member to move forward and backward in an extending direction of the tendon.

According to still another aspect of the disclosure, there is provided a method of operating a surgical member driving apparatus including a surgical member detachably mounted to an endoscope, a cartridge module including a cartridge provided with a base unit coupling with the surgical member at one side, a wire transmitting driving force to the base unit, and a power transmitter coupled to a second end portion of the wire, and a driving module to which the cartridge is detachably coupled, and providing the driving force to the wire, the method including: fastening the base unit to one end portion of the endoscope; coupling the cartridge to the driving module; inserting the one end portion of the endoscope into a body of a patient; controlling operations of the base unit by providing the driving force to the wire; taking the endoscope out of the body of the patient after an operation for the patient is completed; separating the cartridge from the driving module; and separating the base unit from the endoscope, wherein the coupling the cartridge to the driving module is performed before or after the inserting the one end portion of the endoscope into the body of the patient, and wherein the separating the cartridge from the driving module is performed before or after the taking the endoscope out of the body of the patient.

In this case, the method may further include washing the cartridge separated from the endoscope.

In this case, the method may further include discarding the cartridge separated from the endoscope.

The tendon-sheath driving apparatus according to an embodiment of the disclosure includes a power transmitter detachably mounted to a separate driving module, so that the tendon-sheath driving apparatus can be easily replaced and installed before and after an operation.

The surgical member driving apparatus according to an embodiment of the disclosure includes the base unit easily mountable to the endoscope, and thus has an advantage that an operation is performed by coupling various surgical members to the endoscope as necessary.

The surgical member driving apparatus according to an embodiment of the disclosure has advantages in that replacement processes are very quickly and conveniently performed and cost low because the base unit coupled to the endoscope is formed as the cartridge for easy replacement.

Further, the surgical member driving apparatus according to an embodiment of the disclosure employs a unique control module that simulates the positions and operating principles of the base unit and thus supports intuitive control, thereby allowing an operator to easily control the surgical member driving apparatus.

In addition, the surgical member driving apparatus according to an embodiment of the disclosure includes a holder for fastening the endoscope control device to the control module, thereby providing a controlled environment in which one operator can control both the endoscope and the robot arm.

### BRIEF DESCRIPTION OF THE DRAWINGS

FIG. 1 is a perspective view showing that a surgical member driving apparatus is integrated into an endoscope system according to an embodiment of the disclosure.
FIG. 2 is a block diagram of a surgical member driving apparatus according to an embodiment of the disclosure.
FIG. 3 is a perspective view of a cartridge module and a driving module in a surgical member driving apparatus according to an embodiment of the disclosure.
FIG. 4 is an enlarged perspective view of a cartridge of a cartridge module in a surgical member driving apparatus according to an embodiment of the disclosure.
FIG. 5 is a perspective view for describing a process in which a power transmitter provided in the cartridge of FIG. 4 transmits driving force to a wire.
FIG. 6 is an enlarged perspective view of a control module in a surgical member driving apparatus according to an embodiment of the disclosure.
FIG. 7 is an enlarged perspective view of a cartridge module in a surgical member driving apparatus according to an embodiment of the disclosure.
FIG. 8 (a) and (b) and FIG. 9 (a) and (b) are views for describing a first degree of freedom operation in a surgical member driving apparatus according to an embodiment of the disclosure.
FIG. 10 (a) and (b) and FIG. 11 (a) and (b) are views for describing a second degree of freedom operation in a surgical member driving apparatus according to an embodiment of the disclosure.
FIG. 12 (a) and (b) and FIG. 13 (a) and (b) are views for describing a third degree of freedom operation in a surgical member driving apparatus according to an embodiment of the disclosure.
FIG. 14 (a) and (b) and FIG. 15 (a) and (b) are views for describing a fourth degree of freedom operation in surgical member driving apparatus according to an embodiment of the disclosure.
FIGS. 16 and 17 are enlarged perspective views of a cartridge module in a surgical member driving apparatus according to another embodiment of the disclosure.
FIG. 18 is a flowchart of a method of performing an operation using a surgical member driving apparatus according to an embodiment of the disclosure.

### DETAILED DESCRIPTION OF THE INVENTION

Below, embodiments of the present disclosure will be described in detail herein with reference to the accompanying drawings so that this disclosure may be easily performed by a person having ordinary knowledge in the art to which the disclosure pertains. The disclosure may, however, be embodied in many different forms and should not be construed as being limited to the embodiments set forth herein. In the drawings, parts irrelevant to the description are omitted for simplicity of explanation, and like numbers refer to like elements throughout.

In the disclosure, it should be understood that term "include" or "have" indicates that a feature, a number, a step, an operation, an element, a part, or the combination thereof described in the embodiments is present, but does not preclude a possibility of presence or addition of one or more other features, numbers, steps, operations, elements, parts or combinations thereof, in advance.

Further, a wire and a tendon to be described below in the disclosure may refer to the same member with different terms. Likewise, a tube and a sheath may also refer to the same member with different terms.

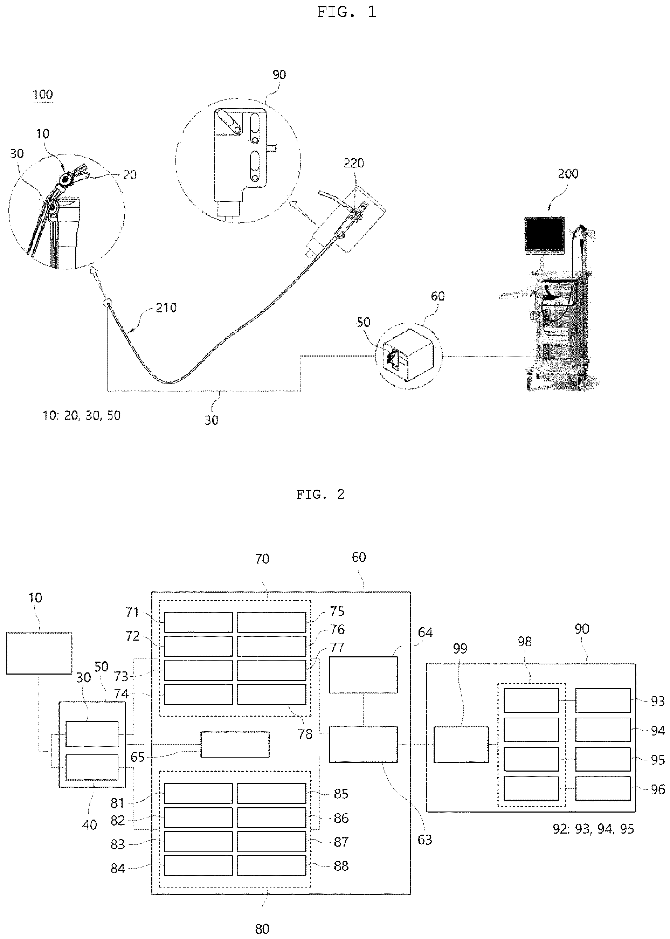

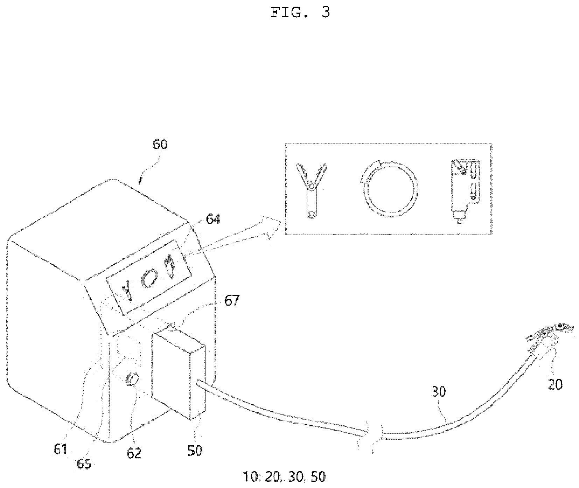

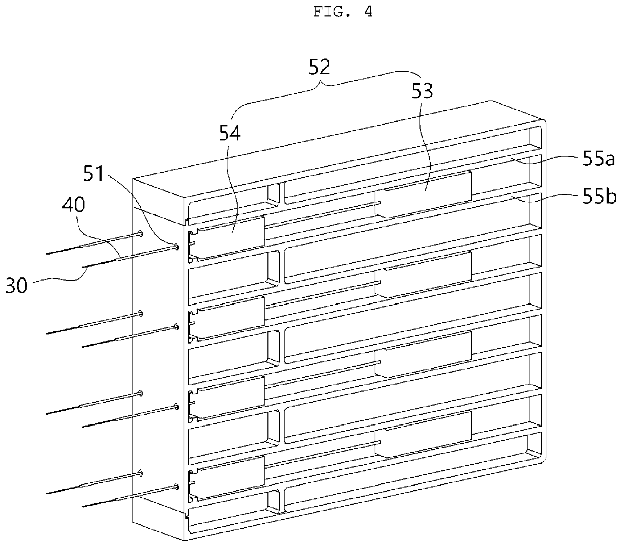

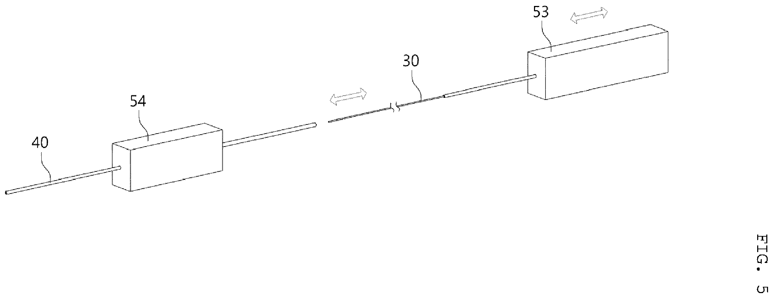

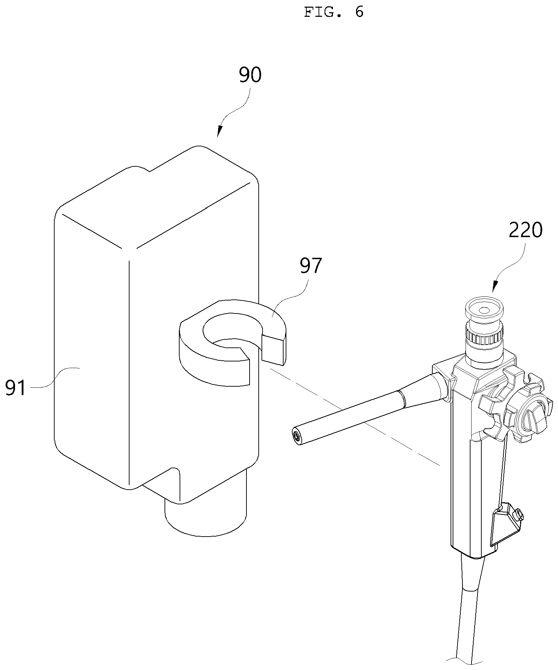

FIG. 1 is a perspective view showing that a surgical member driving apparatus is integrated into an endoscope system according to an embodiment of the disclosure. FIG. 2 is a block diagram of a surgical member driving apparatus according to an embodiment of the disclosure. FIG. 3 is a perspective view of a cartridge module and a driving module in a surgical member driving apparatus according to an embodiment of the disclosure. FIG. 4 is an enlarged perspective view of a cartridge of a cartridge module in a surgical member driving apparatus according to an embodiment of the disclosure. FIG. 5 is a perspective view for describing a process that a power transmitter provided in the cartridge of FIG. 4 transmits driving force to a wire. FIG. 6 is an enlarged perspective view of a control module in a surgical member driving apparatus according to an embodiment of the disclosure.

Referring to FIG. 1, a surgical member driving apparatus 100 according to an embodiment of the disclosure refers to a system for implementing a surgical robot based on an endoscope system 200, in which a base unit 20 is detachably installed in an endoscope 210 of the endoscope system 200, inserted together with the endoscope 210 into a human body, and used to perform physical action, treatment or processing related to the internal organs of the human body.

To this end, the surgical member driving apparatus 100 according to an embodiment of the disclosure includes a cartridge module 10, a driving module 60 configured to drive the cartridge module 10, and a control module 90 configured to receive a driving command input for controlling the driving module 60.

Referring to FIGS. 1 to 4, according to an embodiment of the disclosure, the cartridge module 10 may include the base unit 20, a plurality of wires 30, and a cartridge 50.

First, the cartridge module 10 includes the base unit 20 coupled to a first end portion of the endoscope 210 at which a lens (not shown) is installed. In this case, the base unit 20 refers to a unit in which a surgical member for gripping or excising a part of the tissue in a human body is installed. For example, include a robot arm to perform various operations through the surgical member. In this case, the operations the base unit can perform may include various publicly known operations except grasping or excising a part of tissue.

The base unit 20 may be controlled by driving the plurality of wires 30. To this end, the plurality of wires 30 may include a first end portion coupled to the base unit 20, and a second end portion coupled to the driving module 60 for driving the wires.

According to an embodiment of the disclosure, eight wires 30 may, for example, be provided to operate the base unit 20. In this case, the number of wires 30 in the surgical member driving apparatus 100 according to an embodiment of the disclosure is not limited to eight, but various numbers of wires may be provided as necessary.

Meanwhile, a detailed structure of the base unit 20 and a connection structure of the plurality of wires 30 for operating the base unit 20 will be described later in detail.

Meanwhile, according to an embodiment of the disclosure, the second end portions of the plurality of wires 30 are coupled to the inside of the cartridge 50, so that the cartridge 50 internally provided with the second end portions of the plurality of wires 30 can be connected to the driving module 60.

Referring to FIGS. 3 and 4, the cartridge 50 may be formed as an enclosure having a predetermined volume, and formed with a plurality of insertion holes 51 at one side in which the plurality of wires 30 is insertable.

In addition, the cartridge 50 may be internally provided with a power transmitter 52, to which the second end portion of the wire 30 inserted through the insertion hole 51 is connected. The power transmitter 52 may, as shown in FIG. 4, be shaped like a block formed as sized as accommodatable in the cartridge 50.

In this case, the power transmitter 52 may include a movable member 53 and a supporting member 54.

The movable member 53 refers to a member, to which the end portion of the wire 30 is fastened, and is provided to move forward or backward along a preset path in an inner space of the cartridge 50. Thus, the movement of the movable member 53 may cause an external force to be transmitted to the wire 30.

In more detail, referring to FIGS. 4 and 5, the movable member 53 may move backward in a direction away from the insertion hole 51 to pull the wire 30, and, on the other hand, the movable member 53 may move forward in a direction close to the insertion hole 51 to push the wire 30. Such movement of the movable member 53 is achieved by a driving unit 70 of the driving module 60 (to be described later), and detailed descriptions thereof will be made later.

Meanwhile, to stably guide the movable member 53 to move forward and backward along a preset path, guide portions 55a and 55b may as shown in FIG. 4 be formed protruding to a predetermined height along an extending direction of the wire 30 in an inner space thereof.

In this case, the guide portions 55a and 55b may be positioned at opposite sides of the movable member 53 and form a pair to guide the opposite sides of the movable member 53.

Meanwhile, referring to FIGS. 4 and 5, the wire 30 has an end portion movably supported by the movable member 53 inside the cartridge 50, and a lengthwise body portion between the opposite end portions of the wire may be surrounded by a hollow tube 40. For example, the wires 30 and the tube 40 may be embodied by a publicly known tendon-sheath mechanism.

According to an embodiment of the disclosure, the supporting member 54 refers to a member for holding the tube 40 surrounding the wire 30. Like this, the tube 40 surrounding the wire 30 from outside the wire 30 is held in the supporting member 54, so that relative movement can be generated between the wire 30 moving forward and backward by the movable member 53 and the tube 40.

According to an embodiment of the disclosure, the supporting member 54 surrounding and supporting the wire 30 may be disposed more adjacent to the insertion hole 51 than the movable member 53 holding the end of the wire 30.

According to an embodiment of the disclosure, for example, when eight wires 30 are needed to operate the base unit 20, eight power transmitters 52 may be required to transmit the driving force to the wires 30, respectively.

In this case, as shown in FIG. 4, the plurality of power transmitters 52 may make groups of two inside the cartridge 50, so that four groups of wires can be arranged side by side in up and down directions.

The surgical member driving apparatus according to an embodiment of the disclosure has a structure in which the movable member 53 of the power transmitter 52 moves forward or backward to transmit the driving force to the wire 30, but the structure of the power transmitter 52 is not limited to this structure.

For example, the power transmitter 52 may have a roller structure having an outer circumferential surface, to which the first end portion of the wire is fastened, and is rotatable with respect to a rotary shaft. With this structure, the power transmitter 52 is rotated by the driving unit 70 to transmit the driving force to the wire 30. Alternatively, the wire 30 may be fastened to the power transmitter 52, and the driving unit 70 may directly transmit the driving force to the wire 30.

Meanwhile, the surgical member driving apparatus 100 according to an embodiment of the disclosure includes the driving module 60 to apply the driving force to the wires 30 placed inside the cartridge 50.

Referring to FIG. 3, the driving module 60 includes a housing shaped like an enclosure.

The driving module 60 is internally provided with an accommodating portion 61 in which the cartridge 50 is accommodated, and may be formed with an inlet 67 connecting with the accommodating portion 61 on an outer surface of one side thereof. The accommodating portion 61 formed inside the driving module 60 may be formed as a frame for supporting the cartridge 50 in the state that the cartridge 50 is inserted in the driving module 60.

Further, the cartridge 50 inserted in the driving module through the inlet 67 of the accommodating portion 61 may be seated in and locked to the accommodating portion 61 by a separate holding unit 65.

In this case, the holding unit 65 may be actuated by a motor-based actuator (not shown) and make the cartridge 50 be mechanically locked to or released from the accommodating portion 61.

The driving module 60 may, as shown in FIG. 3, be provided with a button 62 at an outer side portion thereof to control the actuation of the holding unit 65. For example, the button 62 may be pressed to eject the seated and locked cartridge 50 from the accommodating portion 61. The button 62 may include not only a physical button but also a touch-type button activated in a screen displayed on the driving module.

Meanwhile, the holding unit 65 may include a latch or the like publicly known element that is actuated based on an elastic member and locked to a protrusion or groove formed at one side of the cartridge 50.

Meanwhile, the accompanying drawings depict that the accommodating portion 61 is placed inside the driving module 60. Alternatively, the accommodating portion 61 may be formed as opened to the outside of the driving module 60, or may be embodied by any form as long as it can accommodate the cartridge 50 therein.

According to an embodiment of the disclosure, the driving module 60 couples the driving unit 70 to the cartridge 50 in the state that the cartridge 50 is coupled to the accommodating portion 61, thereby applying the driving force to the wires 30 placed inside the cartridge 50.

In this case, the driving unit 70, which applies the driving force to the wires 30 directly or indirectly, may include an electric motor for generating the driving force, and a motor drive for controlling the electric motor based on a signal received from a control unit 63 (to be described later).

According to an embodiment of the disclosure, the driving unit 70 may employ a linear actuator, a rotary actuator, or the like publicly known actuator.

As a concrete example, a first end portion of a piston rod of an electric linear actuator that straightly reciprocates is inserted in a groove portion (not shown) formed in the movable member 53 of the cartridge 50, and makes the movable member 53 move linearly, thereby pulling or pushing the wires 30. The driving force transmitted by this actuation is transmitted to each portion of the base unit 20 to thereby actuate a portion of the base unit 20.

Alternatively, a roller-type power transmitter 52 may be connected to one side of a rotary actuator that rotationally reciprocates is rotated with respect to a predetermined rotary axis, thereby pulling or pushing the wires 30.

Besides the method by which the driving unit 70 indirectly transmits the driving force to the wires 30 via the power transmitter 52, the driving unit 70 may directly transmit the driving force to the wires 30 as the wires 30 are directly connected to the driving unit 70.

Meanwhile, referring to FIG. 2, when the plurality of wires 30 are connected to the cartridge 50, a plurality of driving units 70 may be individually provided to actuate the wires 30. These individual driving units 71 to 78 may include electric motors and motor drives to actuate the motors, respectively. Further, the individual motor drives may respectively receive different commands from the control unit 63 according to operation purposes of the base unit 20.

Referring back to FIG. 2, the driving module 60 according to an embodiment of the disclosure may include a sensor unit 80 for detecting the tension of the plurality of wires 30 included in the cartridge module 10. Thus, the sensor unit 80 may be used to check whether the cartridge module 10 works normally or abnormally in real time, or may be used in various algorithms to enhance the control precision of the robot arm (i.e., the base unit). The sensor unit 80 may include a plurality of sensors 81 to 88, respectively connected to the wires 30.

In this case, the plurality of sensors 81 to 88 in the sensor unit 80 may be properly used by directly detecting the tension of the wire 30 through the movable member 53, measuring the tension (compressive force) of the tube 40 through the supporting member 54, or by measuring a change in the physical properties of the wire 30 and the tube 40 as installed in both the movable and supporting members 53 and 54 and.

Information about the tensions collected as above by the plurality of sensors 81 to 88 in the sensor unit 80 is transmitted to the control unit 63 and used to enhance the control precision as the control unit 63 identifies whether the cartridge module 10 works normally or abnormally based on this information.

Meanwhile, according to an embodiment of the disclosure, the driving module 60 may include the control unit 63 for controlling overall operations of the driving module 60 including the driving unit 70 and the sensor unit 80.

In this case, the control unit 63 may transmit actuation commands to the individual motor drives included in the driving unit 70, and receive information about the tension, etc., of the wire 30 from the sensor unit 80.

Further, the control unit 63 may receive an operator's control command from the control module 90 (to be described later), and transmit the control command as a signal to each driving unit 70.

Meanwhile, the driving module 60 may include a display unit 64 placed at an outer side to display an actuation state of the display unit 20. As shown in FIG. 3, the display unit 64 may visually show how much the positions of the base unit 20 such as an arm portion 23 and forceps 27 of the first rotary portion 22, etc., are rotated. Thus, an operator can check the actuation states of the portions of the base unit 20 in real time, and control the cartridge module 10 more precisely based on the actuation states.

In this case, the display unit 64 may visualize how much the base unit 20 is rotated, based on a control command received from the control module 90, signal information transmitted from the control unit 63 to the driving unit 70, etc.

In this case, the display unit 64 may include a touch screen and directly receive an operator's command through a virtual button displayed on the touch screen.

Meanwhile, information input to the touch screen by an operator may include information for adjusting a control level of the driving unit 70, for example, the sensitivity, etc., of the control module 90 for controlling the driving unit 70. Thus, an operator may set the motion capability of the driving unit 70 according to his/her preferences.

The surgical member driving apparatus 100 according to an embodiment of the disclosure includes the control module 90 allowing an operator to input the control command for controlling the actuation of the base unit 20. In this case, the control module 90 may be connected to the driving module 60 by a wire or wirelessly.

In more detail, referring to FIG. 6, the control module 90 includes a body 91, the volume of which is large enough to be gripped with one hand by an operator. In this case, the body 91 may be internally provided with an angular sensor 98, as shown in FIG. 2, to detect a rotated degree of a control stick 92 (to be described later).

Meanwhile, a holder 97 may be provided at one side of the control module 90 to hold an endoscope control device 220 for the endoscope system 200. With this, the actuation of the endoscope 210 and the base unit 20 may be controlled at once by both hands of one operator. Therefore, the surgical member driving apparatus according to an embodiment of the disclosure has an advantage in that an operator can control the endoscope and the base unit with both hands at once.

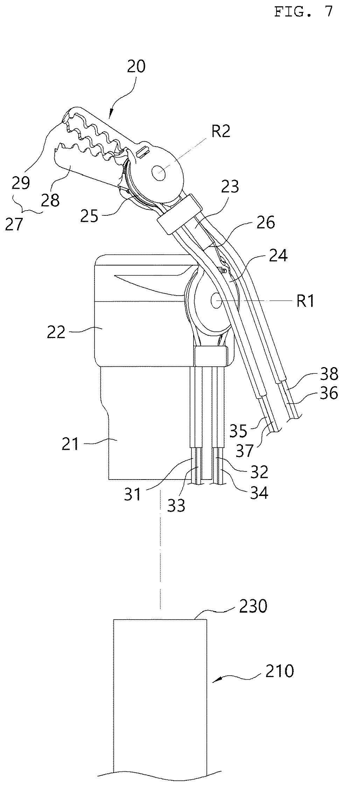

FIG. 7 is an enlarged perspective view of a cartridge module in a surgical member driving apparatus according to an embodiment of the disclosure.

Below, an embodiment of the base unit 20 in a surgical member driving apparatus according to the disclosure will be described in detail with reference to FIG. 7.

According to an embodiment of the disclosure, the base unit 20 includes a base portion 21, the first rotary portion 22, the arm portion 23, and the forceps 27.

The base portion 21 refers to a member for fastening the base unit 20 to an end portion of the endoscope 210, and is internally formed with a hollow in which a first end portion 230 of the endoscope 210 is inserted, thereby coupling with the endoscope 210 while surrounding the outer circumferential surface of the endoscope 210. In this case, the base portion 21 may be stationarily provided not to rotate relative to the endoscope 210.

In addition, the first rotary portion 22 may be disposed outside and coupled to the base portion 21 and rotatable along the outer circumferential surface of the base portion 21. In more detail, the first rotary portion 22 may have an annular shape so that the outer circumferential surface of the base portion 21 and the inner circumferential surface of the first rotary portion 22 can be in contact with each other. In this case, the first rotary portion 22 refers to a member for axially rotating the arm portion 23 (to be described later) along the circumferential direction of the endoscope 210.

According to an embodiment of the disclosure, the arm portion 23, which refers to a lengthwise member extended to a predetermined length along the extending direction of the endoscope, may be rotatably coupled to one side of the outer circumferential surface of the first rotary portion 22. In other words, a first end portion 24 of the arm portion 23 may be coupled to the first rotary portion 22 so as to clockwise or counterclockwise pivot around a first rotary axis R1 shown in FIG. 7. As necessary, the arm portion 23 may further include a bending portion 26 bent at a predetermined angle toward the inside of the endoscope 210 as shown in FIG. 7.

Further, the forceps 27 including a first gripper 28 and a second gripper 29 may be rotatably coupled to the second end portion 25 of the arm portion 23. In this case, both the first gripper 28 and the second gripper 29 may be coupled so as to clockwise or counterclockwise pivot around a second rotary axis R2.

In this case, the first gripper 28 and the second gripper 29 may rotate independently of each other and interact to grip a part of the tissue in a human body or release the gripped part.

According to an embodiment of the disclosure, the cartridge module 10 includes the wires 30 connected to the portions of the base unit 20 and transmitting the driving force to the portions of the base unit 20.

In this case, the wires 30 may be pulsed or pushed by the driving force applied by the driving module 60 (to be described later), thereby actuating the portions connected to the wires 30 in the base unit 20.

In more detail, the wires 30 may include a first wire 31 and a second wire 32 to clockwise or counterclockwise rotate the first rotary portion 22 with respect to the base portion 21. In this case, the first wire 31 and the second wire 32 may be fastened to the first rotary portion 22 or the base portion 21, so that the first rotary portion 22 and the base portion 21 can rotate relative to each other by the driving force.

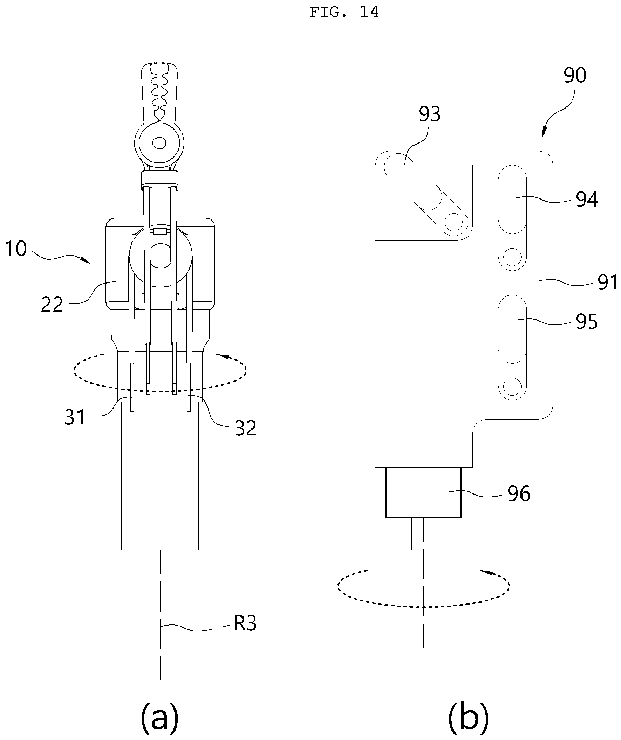

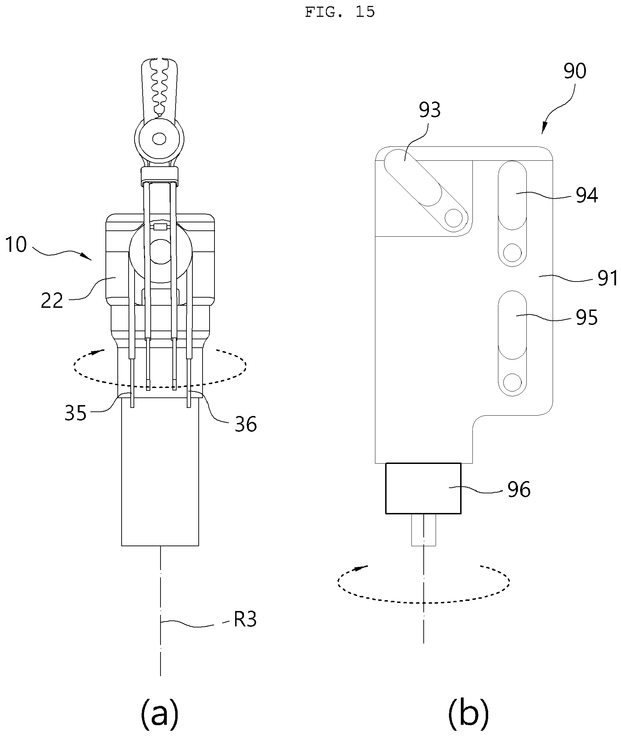

As a concrete example, when the first wire 31 is pulled or the second wire 32 is pushed, the first rotary portion 22 may rotate counterclockwise along the outer circumferential surface of the base portion 21 with respect to a central axis R3 passing through the center the base portion 21. On the other hand, when the second wire 32 is pulled or the first wire 31 is pushed, the first rotary portion 22 may rotate clockwise. Thus, the arm portion 23 fastened to the first rotary portion 22 may also move to a destination along the circumferential direction of the endoscope 210 (see FIGS. 14 and 15)

Meanwhile, the wires 30 may include a third wire 33 and a fourth wire 34 to clockwise or counterclockwise rotate the arm portion 23 with respect to the first rotary axis R1. In this case, the third wire 33 and the fourth wire 34 may be fastened to the first end portion 24 of the arm portion 23, and wound around a wire guide formed in the first end portion 24 of the arm portion 23 as shown in FIG. 7.

In this case, when the third wire 33 is pulled or the fourth wire 34 is pushed, the arm portion 23 may pivot counterclockwise. On the other hand, the fourth wire 34 is pulled or the third wire 33 is pushed, the arm portion 23 may pivot clockwise. Thus, the forceps 27 fastened to the arm portion 23 may rotate and reach a position desired by an operator.

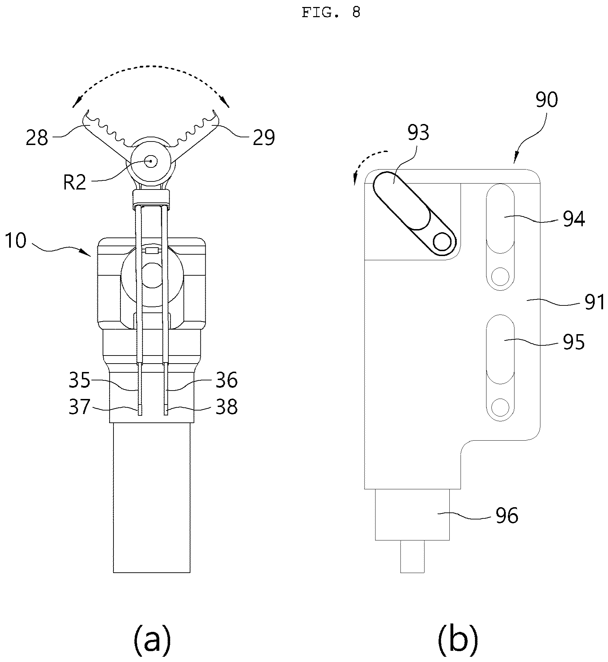

Meanwhile, the wires 30 may include a fifth wire 35 and a sixth wire 36 to make the first gripper 28 clockwise or counterclockwise pivot around the second rotary axis R2. In this case, the fifth wire 35 and the sixth wire 36 may be fastened to one side of the first gripper 28 (see FIGS. 8 to 11)

In addition, the wires 30 may include a seventh wire 37 and an eighth wire 38 to make the second gripper 29 clockwise or counterclockwise pivot around the second rotary axis R2. The seventh wire 37 and the eighth wire 38 may also be fastened to one side of the second gripper 29.

In this case, the principle that the first gripper 28 and the second gripper 29 pivot clockwise or counterclockwise around the second rotary axis R2 is similar to the principle of rotating the arm portion 23, and thus repetitive descriptions thereof will be omitted.

Meanwhile, the first wire 31 and the second wire 32 may be independent of each other. Alternatively, the first wire 31 and the second wire 32 may be connected to each other generally forming a single wire, or may respectively refer to one wire and the other wire divided from the wire at a fixed point.

Similarly, the third wire 33 and the fourth wire 34, the fifth wire 35 and the sixth wire 36, and the seventh wire 37 and the eighth wire 38 may also be wires independent of one another or connected to each other forming a single wire like the first wire 31 and the second wire 32.

According to an embodiment of the disclosure, the base unit 20 may be actuated broadly having four degrees of freedom by the wires 30. In more detail, four degrees of freedom may include an operation of closing or opening the first gripper 28 and the second gripper 29 of the forceps 27 to grip tissue (hereinafter, referred to as a 'first degree of freedom operation'), an operation of pivoting the whole forceps 27 around the second rotary axis R2 (hereinafter, referred to as a 'second degree of freedom operation'), an operation of pivoting the arm portion 23 around the first rotary axis R1(hereinafter, referred to as a 'third degree of freedom operation'), and an operation of axially rotating the first rotary portion 22, to which the arm portion 23 is fastened, along the outer circumferential surface of the endoscope 210 (hereinafter, referred to as a 'fourth degree of freedom operation'). Below, the degrees of freedom operations that the robot arm (i.e., the base unit) actuated by the surgical member driving apparatus according to an embodiment of the disclosure has, and control of the control module for the degrees of freedom operations will be described with reference to other accompanying drawings.

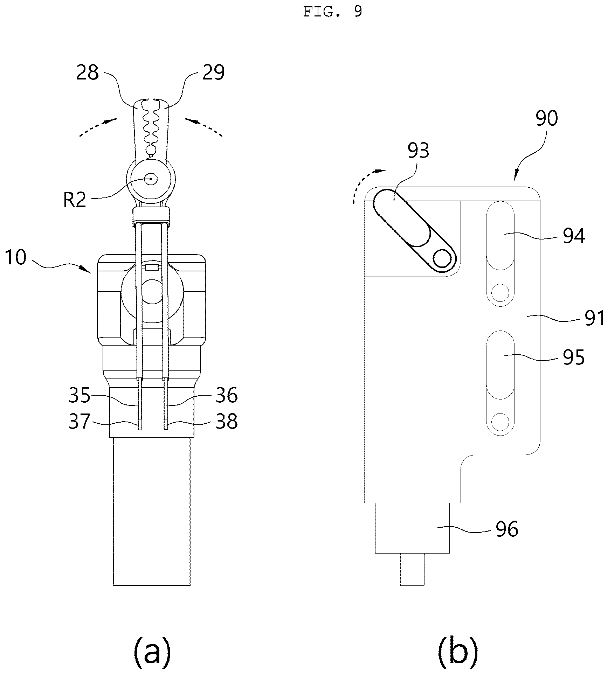

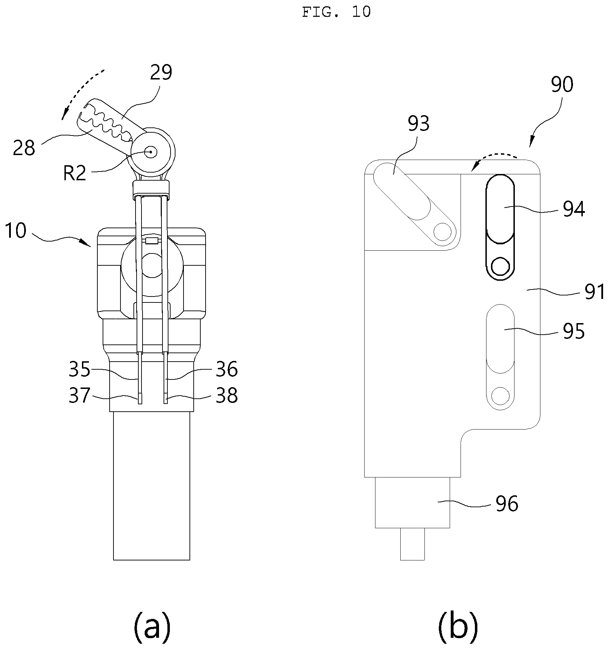

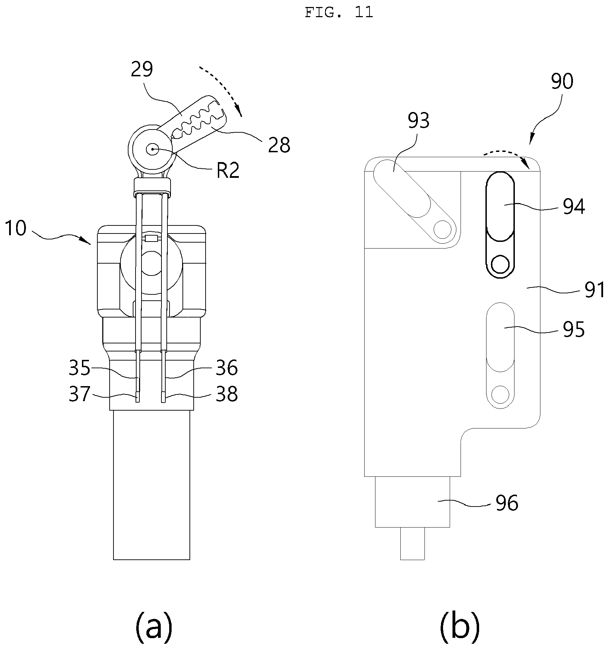

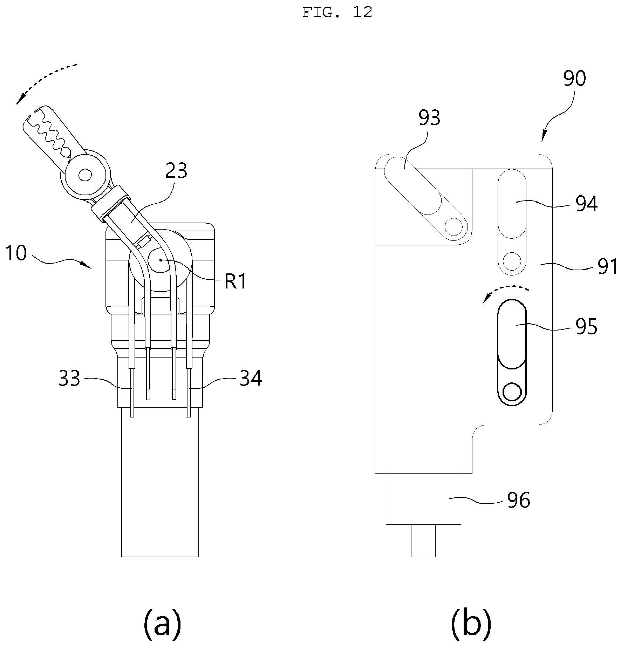

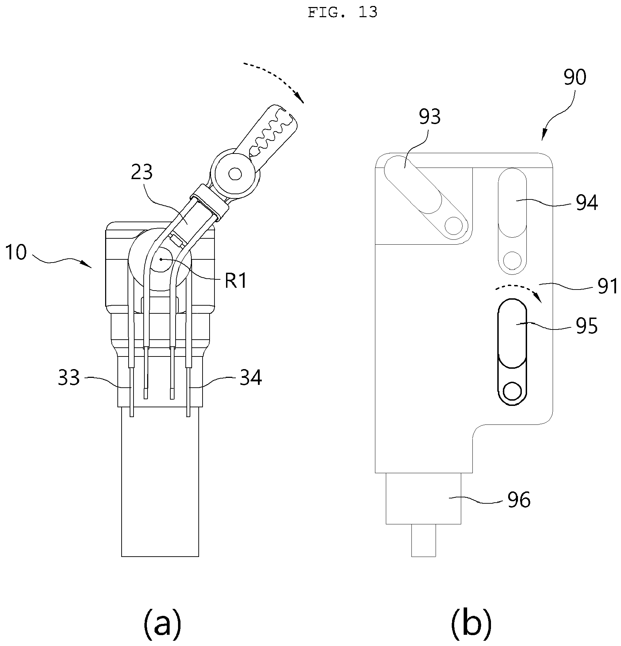

FIG. 8 (a) and (b) and FIG. 9 (a) and (b) are views for describing a first degree of freedom operation in a surgical member driving apparatus according to an embodiment of the disclosure. FIG. 10 (a) and (b) and FIG. 11 (a) and (b) are views for describing a second degree of freedom operation in a surgical member driving apparatus according to an embodiment of the disclosure. FIG. 12 (a) and (b) and FIG. 13 (a) and (b) are views for describing a third degree of freedom operation in a surgical member driving apparatus according to an embodiment of the disclosure. FIG. 14 (a) and (b) and FIG. 15 (a) and (b) are views for describing a fourth degree of freedom operation in surgical member driving apparatus according to an embodiment of the disclosure. In FIGS. 8 to 15, (a) illustrates the operations of the base unit, and (b) illustrates the controlled state of the control module for operating the base unit.

Referring to FIGS. 2 and 8, the control stick 92 may be provided at one side of the body 91.

In this case, the control stick 92 refers to a means for allowing an operator to input a control command to operate the portions of the based unit 20. The wires 30 are fastened and may, for example, be shaped like a bar extended to a predetermined length. In this case, the shape and structure of the control stick 92 may be variously modified.

According to an embodiment of the disclosure, the control stick 92 may clockwise or counterclockwise pivot around the rotary shaft at a predetermined angle.

Such pivoting of the control stick 92 simulates pivoting of each portion in the base unit 20, and therefore the control stick 92 may pivot in the same direction as or in the opposite direction to a portion of the base unit to which the wire 30 is fastened. Therefore, an operator can more intuitively control the operations of the base unit 20.

According to an embodiment of the disclosure, the pivoted angle of the control stick 92 may be detected by the angular sensor 98 installed in the control module 90, and the driving module 60 may be actuated corresponding to the pivoted angle of the control stick 92 detected as above, thereby operating the base unit 20 as the tension of the wires 30 is adjusted based on the actuation of the driving module 60.

In this case, according to an embodiment of the disclosure, as shown in FIGS. 8 and 9, a first control stick 93 may be used to pivot the first gripper 28 and the second gripper 29 around the second rotary axis R2 in directions opposite each other. Therefore, the first degree of freedom operation is implemented so that the forceps 27 can grip a part of the tissue in a human body, or release the gripped part.

In more detail, as shown in FIG. 8, when the first control stick 93 pivots counterclockwise, the first gripper 28 and the second gripper 29 may pivot so that their distal ends can move away from each other (i.e., be opened). On the other hand, when the first control stick 93 pivots clockwise as shown in FIG. 9, the first gripper 28 and the second gripper 29 may pivot so their distal ends can move close to each other.

According to an embodiment of the disclosure, the first control stick 93 is simply changed only in the pivoting direction to implement the first degree of freedom operation for closing and opening the forceps 27, so that an operator can control the forceps 27 without difficulties.

Meanwhile, a second control stick 94 may be used to pivot the first gripper 28 and the second gripper 29 in the same direction to implement the second degree of freedom operation for pivoting the whole forceps 27 around the second rotary axis R2 as shown in FIGS. 10 and 11. Such pivoting of the first gripper 28 and the second gripper 29 based on the second control stick 94 may move the whole movable range of the forceps 27.

In this case, the pivoting direction of the second control stick 94 may be set to be the same as the pivoting direction of the forceps 27. Thus, an operator can intuitively pivot the forceps 27.

Meanwhile, referring back to FIGS. 8 to 11, the pivoting of the first control stick 93 and the second control stick 94 is converted into the pivoting of the first gripper 28 and the second gripper 29 as follows.

The first gripper 28 may pivot in the same direction as the pivoting direction of the first control stick 93 and the second control stick 94. Further, the second gripper 29 may pivot in the opposite direction to the pivoting direction of the first control stick 93, but may pivot in the same direction as the pivoting direction of the second control stick 94.

In the surgical member driving apparatus 100 according to an embodiment of the disclosure, only two simple control sticks 92 are enough to easily implement not only the gripping operation of the forceps but also the expansion of the movable range around the second rotary axis.

Meanwhile, referring to FIGS. 12 and 13, a third control stick 95 may be used to implement the third degree of freedom operation. In other words, the arm portion 23 may pivot around the first rotary axis R1 in the same direction as the pivoting direction of the third control stick 95. In this case, the pivoting direction of the third control stick 95 may also be set to be the same as the pivoting direction of the arm portion 23 for intuitive control of an operator like the second control stick 94.

Meanwhile, according to an embodiment of the disclosure, the control module 90 may include a control handle 96 disposed in an upper or lower portion of the body 91 and axially rotating with respect to a rotary axis perpendicular to the ground, in addition to the control stick 92.

In more detail, referring to FIGS. 14 and 15, the control handle 96 may have a circular outer circumferential surface. By rotating the control handle 96, it is possible to implement the fourth degree of freedom operation for axially rotating the first rotary portion 22 of the cartridge module 10 in the same direction as the axial rotating direction of the control handle 96 at the outside of the endoscope 210.

Unlike the control stick 92, the control handle 96 does not pivot but axially rotates, and thus corresponds to the axial rotation of the first rotary portion 22 rotating along the outer circumferential surface of the endoscope 210, so that an operator can more intuitively control the base unit.

The control stick 92 and the control handle 96 may be positioned in such a manner that the first control stick 93 - the second control stick 94 - the third control stick 95 - the control handle 96 are arranged in order from the top to the bottom of the control module when viewed in FIG. 8.

Such arrangement corresponds to a manner that the portions of the base unit 20 are arranged in the order of the forceps 27 - the arm portion 23- the first rotary portion 22 with respect to a height direction to the ground, so that an operator can intuitively and quickly which of the control sticks or the control handle corresponds to the portion of the base unit 20 even without recognizing a relationship between the elements of the control module 90 and the portions of the base unit 20.

Besides, the control module 90 may further include a control button 99 to generate a control command when pressed by external force. In this case, the control button 99 may be embodied by a publicly known physical button.

Meanwhile, in the surgical member driving apparatus according to an embodiment of the disclosure, the base unit 20 shown in FIG. 7 is merely an example of the base unit 20 employable by the surgical member driving apparatus 100 according to an embodiment of the disclosure. In other words, the base unit applicable to the surgical member driving apparatus 100 according to an embodiment of the disclosure is not limited to that of this embodiment, but various base units may be employed for the surgical member driving apparatus according to the disclosure.

In this regard, other base units 20' and 20" applicable to the surgical member driving apparatus 100 according to an embodiment of the disclosure will be described with other accompanying drawings. In this case, descriptions about the base portion 21, the first rotary portion 22, the arm portion 23 and the like overlapping elements will be replaced by the foregoing descriptions thereof to avoid repetitive descriptions.

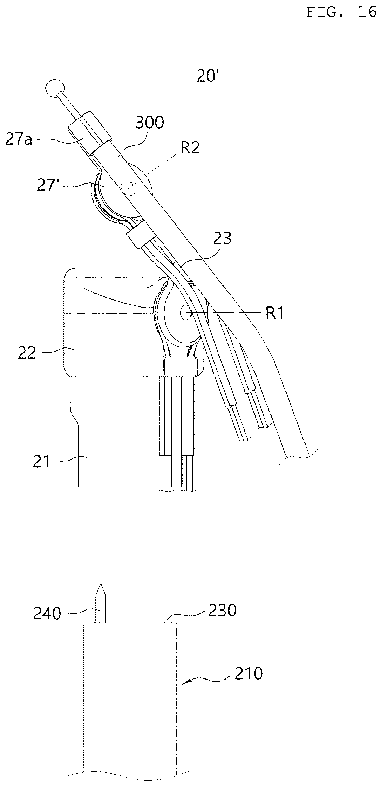

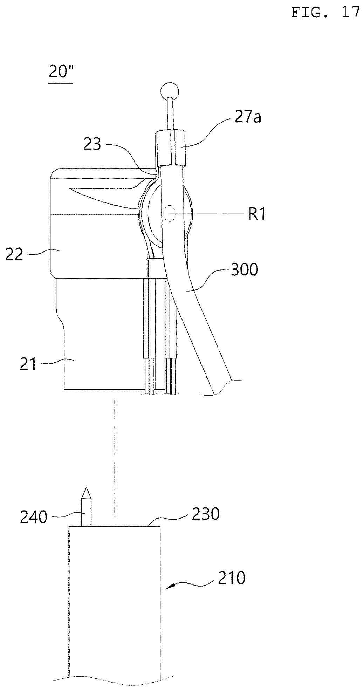

FIGS. 16 and 17 are enlarged perspective views of a cartridge module in a surgical member driving apparatus according to another embodiment of the disclosure.

First, referring to FIG. 16, according to an embodiment of the disclosure, the base unit 20' may be formed excluding the first gripper 28 and the second gripper 29 from the foregoing base unit 20 including the forceps 27. In other words, the base unit 20' may include the base portion 21, the first rotary portion 22 and the arm portion 23, and a second rotary portion 27' instead of the first gripper 28 and the second gripper 29 may be coupled to the second end portion of the arm portion 23 as shown in FIG. 16.

In this case, a separate surgical member 300 may be fastened to one side of the second rotary portion 27' instead of the forceps 27 to apply external force to the tissue of a human body. In this regard, the separate surgical member 300 fastened to the second rotary portion 27' refers to a surgical member conventionally used as integrated with the endoscope of the endoscope system 200, and may for example include, but not limited to, surgical forceps, an endoscopic submucosal dissection (ESD) knife, a surgical snare, a surgical basket, a surgical net, a surgical clip, surgical micro-scissors, and so on. However, such separate surgical members 300 listed above are merely examples, and various other surgical members may be fastened to the second rotary portion.

In this case, the surgical member 300 is connected to the driving module 60 of the surgical member driving apparatus 100 according to an embodiment of the disclosure by the wires, and thus receives the driving force from the driving module 60. In this case, the principle of receiving the driving force through the wires is the same or similar to the principle that the first rotary portion 22 and the arm portion 23 receive the driving force through the wires, and therefore repetitive descriptions thereof will be omitted.

Alternatively, the surgical member 300 may receive the driving force from an additional driving module mechanically separated from the surgical member driving apparatus 100 according to an embodiment of the disclosure.

In this case, a control means for controlling the operations of the surgical member 300 may, for convenience, be detachably fastened to the control module 90 of the surgical member driving apparatus 100 according to an embodiment of the disclosure, like the endoscope control device 220.

Meanwhile, the separate surgical member 300 may be fastened by a fastening member 27a formed at one side of the second rotary portion 27'. As a concrete example, the fastening member 27a may have an annular shape to surround one side of the outer circumference of the separate surgical member 300 as shown in FIG. 15. Further, the fastening member 27a may be made of a resilient material to prevent the surgical member 300 from being easily separated, thereby applying pressure while surrounding the outer circumferential surface of the surgical member 300. However, the fastening member 27a shown in the accompanying drawing is merely an example, and may have any shape as long as it can easily support the surgical member 300.

Meanwhile, the second rotary portion 27' may pivot around the second rotary axis R2, like the first gripper 28 and the second gripper 29. As the end portion of the surgical member 300 pivots in a desired direction, a medical person can more effectively perform an operation.

Like this, when the base unit 20' is formed to support and hold the separate surgical member 300, a medical person can add the separate surgical member 300 in addition to the surgical member 240 provided in the existing endoscope system 200 and thus perform a more complex and three-dimensional operation within a single endoscopic procedure.

In particular, the separate surgical member 300 has an advantage in that its position is freely changeable through the operations of the first rotary portion 22, the arm portion 23 and the second rotary portion 27' as compared with the surgical member 240 provided in the existing the endoscope system 200.

In addition, the surgical member driving apparatus 100 according to an embodiment of the disclosure has an advantage in that the function of the existing endoscope system 200 is expandable because a medical person can select various surgical members 300 according to purposes.

Meanwhile, referring to FIG. 17, as another example related to the base unit, the base unit 20" may be formed excluding the foregoing second rotary portion 27'.

In other words, the base unit 20" may be formed including the base portion 21 and the arm portion 23 among the foregoing elements of the base unit 20, and including the fastening member 27a for fastening the separate surgical member 300 to one side of the arm portion 23 as shown in FIG. 17.

In this case, the second rotary portion 27' is absent as compared with that of the foregoing base unit 20', and therefore pivoting at the end portion of the surgical member 300 may be partially restricted. However, the absence of the second rotary portion 27' makes it possible to remove the wires, thereby having an advantage of generally simplifying the apparatus.

Although the second rotary portion 27' is absent, the axial rotation of the first rotary portion 22 and the pivoting of the arm portion 23 around the first rotary axis R1 are still possible, thereby sufficiently securing the movable range of the surgical member 300.

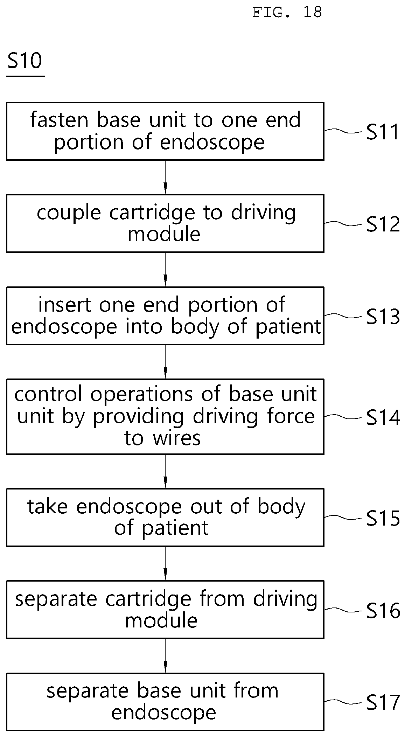

FIG. 18 is a flowchart of a method of performing an operation using a surgical member driving apparatus according to an embodiment of the disclosure.

Referring to FIG. 18, a method 10 of performing an operation using a surgical member driving apparatus according to an embodiment of the disclosure is as follows.

First, to use the surgical member driving apparatus 100 according to an embodiment of the disclosure, an operator couples the base unit 20 to the first end portion of the endoscope 210 (S11). Further, an operator inserts the cartridge 50 in the accommodating portion of the driving module 60 to thereby couple the driving module and the cartridge (S12). In this regard, operation S11 of coupling the base unit to the endoscope and operation S12 of coupling the cartridge to the accommodating portion do not have a fixed precedence order, and any of them may be performed first according to an operator's preference.

In addition, an operator couples the control module 90 to the endoscope control device 220, and then inserts the first end portion of the endoscope 210, to which the base unit 20 is installed, into a body of a patient (S13).

However, alternatively, an operator may insert the first end portion of the endoscope 210 provided with the base unit 20 into the body of the patient (S13), and then couple the cartridge and the driving module (S12). In this case, the endoscope is inserted in the body in the state that the cartridge and the driving module are separated from each other, thereby having an advantage in that the effect of the external force from the wire included in the base unit 20 on the insertion of the endoscope is minimized.

For example, to excise an affected part of a patient, an operator may use the control module 90 to control the operations of the forceps or the surgical member 300 of the base unit 20 installed at the end portion of the endoscope 210 (S14).

In this case, an operator may perform an operation while checking the operations of the base unit 20 in real time through an image obtained by the endoscope. In other words, an operator may control the control module 90 while grasping the positions of the robot arm (i.e., the base unit) and the surgical member through an endoscopic screen, and it is therefore possible to immediately make feedback related to the precision of the control.

After the operation is completed, an operator takes out the endoscope 210 from the body of the patient (S15).

Then, an operator may take the cartridge 50 out of the accommodating portion of the driving module 60 (S16), and separate the base unit 20 from the end portion of the endoscope (S17). Even in this case, operation S16 of taking the cartridge out of the driving module and operation S17 of separating the base unit from the endoscope do not have a fixed precedence order.

Meanwhile, alternatively, an operator may first separate the cartridge 50 from the driving module 60 (S16) before taking the endoscope 210 out of the body of the patient (S15). When the cartridge 50 is first separated from the driving module 60, the effect of the external force from the wire of the base unit 20 on the taking-out of the endoscope may be minimized.

In this case, the cartridge module 10 of the surgical member driving apparatus 100 according to an embodiment of the disclosure may be discarded after one use. Thereafter, when another operation using the surgical member driving apparatus 100 is additionally needed, an operator mounts a new cartridge module 10 to the endoscope 210 and performs the operation.

Alternatively, the cartridge module 10 may be discarded after several uses. In this case, a washing process using an additional washing solution may be applied to the cartridge module 10 used once in operation. Further, when the cartridge module 10 is used several times, an additional measuring member (not shown) for counting the number of uses may be provided inside the cartridge and forcibly prevent the cartridge module 10 from being used more than a predetermined number of times.

As described above, the surgical member driving apparatus according to an embodiment of the disclosure may employ the cartridge module easily mountable to the endoscope, and the control module supporting an intuitive interface, thereby introducing an economical and homogeneous surgical robot based on the functions of the endoscope.

The surgical member driving apparatus according to an embodiment of the disclosure includes the cartridge detachably mounted to the driving module, so that a consumable cartridge module can be easily replaced.

In more detail, the cartridge module is required to be replaced after one use or several uses due to factors of hygiene or durability. The surgical member driving apparatus according to an embodiment of the disclosure has advantages in that replacement is quickly and conveniently performed and the help of experts is not required because the cartridge module is replaced by simply separating the base unit and the cartridge from the endoscope and the driving module and coupling a new base unit and a new cartridge to the endoscope and the driving module.

In addition, the surgical member driving apparatus according to an embodiment of the disclosure employs a unique control module that simulates the operation of the base unit and thus supports intuitive control, thereby allowing an operator to control the surgical member driving apparatus easily.

Further, the surgical member driving apparatus according to an embodiment of the disclosure employs the holder for fastening the endoscope control device to the control module, thereby providing a controlled environment in which one operator can control both the endoscope and the base unit.

Although an embodiment of the disclosure has been described above, the spirit of the disclosure is not limited to the foregoing embodiments, those skilled in the art and understanding the spirit of the disclosure may easily suggest other embodiments by adding, modifying, deleting, supplementing, etc., elements without departing from the scope of the disclosure, and such embodiments also fall within the scope of the disclosure.

**DESCRIPTION OF REFERENCE NUMERALS**

| | | | |
|---|---|---|---|
| 10 | cartridge module | 20 | base unit |
| 30 | wire(tendon) | 40 | tube(sheath) |
| 50 | cartridge | 60 | driving module |
| 63 | control unit | 64 | display |
| 70 | driving unit | 80 | sensor unit |
| 90 | control module | | |
| 100 | surgical member driving apparatus | | |
| 200 | the endoscope system | | |

## Claims

1. A surgical member driving apparatus for operating a surgical member detachably mounted to an endoscope, comprising:
a cartridge module comprising a base unit fastened to one end portion of the endoscope and coupling with the surgical member at one side, a wire comprising a first end portion coupled to the base unit and transmitting driving force to the base unit, and a cartridge provided with a power transmitter coupled to a second end portion of the wire; and
a driving module comprising an accommodating portion to which the cartridge is detachably coupled, and providing the driving force to the wire of the cartridge module coupled to the accommodating portion.

2. The surgical member driving apparatus of claim 1, wherein the base unit comprises:
a base portion to be fastened to the one end portion of the endoscope; and
a first rotary portion rotatably coupled to the base portion with respect to an extending direction of the endoscope.

3. The surgical member driving apparatus of claim 2, wherein
the wires form a pair, and
the pair of wires comprises a first wire and a second wire to rotate the first rotary portion clockwise or counterclockwise with respect to the extending direction of the endoscope.

4. The surgical member driving apparatus of claim 2, further comprising:
an arm portion extended in the extending direction of the endoscope, and coupled to the first rotary portion pivotally with respect to a first rotary axis perpendicular to the extending direction of the endoscope, and
a third wire and a fourth wire to pivot the arm portion around the first rotary axis.

5. The surgical member driving apparatus of claim 4, wherein the arm portion comprises a fastening member to fasten the surgical member to one side of the arm portion.

6. The surgical member driving apparatus of claim 4, further comprising a second rotary portion coupled to an end portion of the arm portion pivotally with respect to a second rotary axis perpendicular to the extending direction of the arm portion.

7. The surgical member driving apparatus of claim 6, wherein the surgical member comprises forceps provided in the second rotary portion pivotally with respect to the second rotary axis and comprising a first gripper and a second gripper.

8. The surgical member driving apparatus of claim 6, wherein the second rotary portion comprises a fastening member to fasten the surgical member to the second rotary portion.

9. The surgical member driving apparatus of claim 1, wherein the surgical member comprises one among surgical forceps, an endoscopic submucosal dissection (ESD) knife, a surgical snare, a surgical basket, a surgical net, a surgical clip, surgical micro-scissors.

10. The surgical member driving apparatus of claim 1, wherein the driving module comprises a holding unit to mechanically couple the cartridge seated in the accommodating portion to the accommodating portion.

11. The surgical member driving apparatus of claim 1, further comprising a control module to input a control command for controlling operations of the surgical member or the base unit through the driving module.

12. The surgical member driving apparatus of claim 11, wherein
the control module comprises:
a body; and
a control stick or a control button pivotally provided on the body, and
the wire is actuated as the control stick is pivoted or the control button is pressed.

13. A tendon-sheath driving apparatus for driving a tendon and a sheath, comprising:
a supporting member to which at least one side of the sheath is fastened;
a movable member coupled to one side of the tendon to move the tendon relative to the sheath; and
a driving module mechanically coupled to the movable member and moving or rotating the movable member,
wherein the movable member is detachably coupled to the driving module.

14. The tendon-sheath driving apparatus of claim 13, wherein
the driving module comprises a linear actuator coupled to the movable member and making the movable member move rectilinearly, and
the tendon moves relative to the sheath by the rectilinear movement of the movable member.

15. The tendon-sheath driving apparatus of claim 13, wherein the supporting member and the movable member are formed as a single body to be replaced and discarded after one or more uses.

16. The tendon-sheath driving apparatus of claim 13, wherein
the tendon comprises a plurality of tendons,
the supporting member and the movable member respectively comprise a plurality of supporting members and a plurality of moveable members to correspond to the plurality of tendons, and
the plurality of movable members is installed in a cartridge shaped like an enclosure, and integrally coupled to the driving module.

17. The tendon-sheath driving apparatus of claim 16, wherein the cartridge comprises a guide portion at one side thereof to guide the movable member to move forward and backward in an extending direction of the tendon.

18. A method of operating a surgical member driving apparatus comprising a surgical member detachably mounted to an endoscope, a cartridge module comprising a base unit coupling with the surgical member at one side, a wire transmitting driving force to the base unit, and a cartridge provided with a power transmitter coupled to a second end portion of the wire, and a driving module to which the cartridge is detachably coupled, and providing the driving force to the wire, the method comprising:
fastening the base unit to one end portion of the endoscope;
coupling the cartridge to the driving module;
inserting the one end portion of the endoscope into a body of a patient;
controlling operations of the base unit by providing the driving force to the wires;
taking the endoscope out of the body of the patient after an operation for the patient is completed;
separating the cartridge from the driving module; and
separating the base unit from the endoscope,
wherein the coupling the cartridge to the driving module is performed before or after the inserting the one end portion of the endoscope into the body of the patient, and
wherein the separating the cartridge from the driving module is performed before or after the taking the endoscope out of the body of the patient.

19. The method of claim 18, further comprising washing the cartridge separated from the endoscope.

20. The method of claim 18, further comprising discarding the cartridge separated from the endoscope.
